# EUROPEAN PATENT APPLICATION

(11) **EP 3 988 542 A1**
(43) Date of publication of application: **27.04.2022**
(21) Application number: 20826388.9
(22) Date of filing: 19.06.2020
(51) Int. Cl.: C07D 239/48, C07D 495/04, C07D 401/12, C07D 403/12, A61K 31/505, A61K 31/519, A61K 31/506, A61P 35/00

(54) **PYRIMIDINE DERIVATIVE INHIBITING GROWTH OF CANCER CELL AND MEDICINAL USE THEREOF**

(30) Priority: 20.06.2019 KR 20190073345
(71) Applicant: Oncobix Co.Ltd., Giheung-u Yongin-si, Gyeonggi-do 16950 (KR)
(72) Inventor: RYU, Hyung-Chul, Osan-Si, Gyeonggi-do 18113 (KR); KIM, Jae-Sun, Suwon-Si, Gyeonggi-do 16658 (KR); KIM, Sung-Eun, Seoul 05688 (KR); LEE, Sun-Ho, Seoul 06571 (KR); LEE, Yong-Hyub, Yongin-si, Gyeonggi-do 16948 (KR)
(74) Representative: J A Kemp LLP
(86) International application number: PCT/KR2020/007982
(87) International publication number: WO 2020/256477

(57) **Abstract**

The present invention provides novel pyrimidine derivative compounds inhibiting growth of cancer cells and pharmaceutically acceptable salts thereof. The present invention provides a pharmaceutical composition comprising these compounds or pharmaceutically acceptable salts. The present invention also provides a medical use for treatment of lung cancer, characterized by using these pyrimidine derivative compounds and pharmaceutically acceptable salts thereof as an active ingredient. The present invention also provides a method for treatment of lung cancer comprising administering an effective dose of compounds according to the present invention, salts thereof or a composition comprising them.

## Description

### [TECHNICAL FIELD]

The present invention relates to pyrimidine derivative compounds effective for inhibiting growth of cancer cells and anti-cancer uses thereof.

### [BACKGROUND ART]

Various mutations in the kinase domain of epidermal growth factor receptor (EGFR) are found as oncogenic genes in patients with non-small cell lung cancer, and Gefitinib, Erlotinib, Osimertinib, and the like are used as a small molecule kinase inhibitor for the epidermal growth factor receptor (EGFR) to treat them.

Among them, lung cancer caused by an insertion in exon 20 of EGFR mutation (EGFR EXON 20 Insertion mutation) accounts for 5% of all mutations, and various epidermal growth factor receptor inhibitors (EGFR inhibitors) have been evaluated for its treatment, but there is still no drug approved as a standard therapy for treatment of EGFR EXON 20 Insertion mutation lung cancer, so there is an urgent need to develop a drug that can treat this.

### [DISCLOSURE]

### [TECHNICAL PROBLEM]

Accordingly, a problem to be solved by the present invention is to provide compounds useful for treatment of lung cancer, particularly, lung cancer showing epidermal growth factor receptor mutation properties and medicinal uses thereof.

### [TECHNICAL SOLUTION]

To solve the above problem, the present invention provides a compound of Chemical formula 1 below or a pharmaceutically acceptable salt thereof:

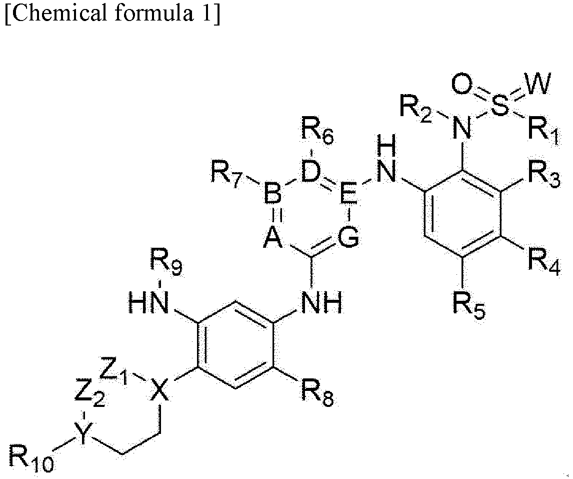

In the Chemical formula 1,
A, B, D, E, and G are combinations of A=N, B=C, D=C, E=C, and G=N, or combinations of A=N, B=C, D=N, E=C, and G=C,
W is oxygen or NH,
X and Y are each independently CH, oxygen or nitrogen,
Z₁ and Z₂ are each independently not present when X or Y is oxygen, or are each independently C1 to C4 alkyl, or are each independently composed of carbon atom and are linked to each other to form a 5-, 6- or 7-membered ring together with X and Y, when X or Y is not oxygen,
R₁ is C1 to C4 alkyl,
R₂ is hydrogen, or C1 to C4 alkyl,
R₃ and R₄ are each independently hydrogen, halogen, OH, OMe, OEt, CN, CF₃, C1 to C4 alkyl, or are linked to each other to form a 5- or 6-membered (fused to phenyl) heteroaryl ring, and
R₅ is hydrogen, halogen, OH, OMe, OEt, CN, CF₃, or C1 to C4 alkyl,
R₆ and R₇ are each independently hydrogen, halogen, OH, OMe, OEt, CN, CF₃, COOH, COO-C₁₋₄ alkyl, COO-C₁₋₅ cycloalkyl, C1 to C4 alkyl, or are linked to each other to form a 5- or 6- membered heteroaryl ring,
R₈ is hydrogen, halogen, OH, OMe, OEt, CN, CF₃, or C1 to C4 alkyl,
R₉ is -C(O)-CR=CR₂ or C1 to C4 alkyl, and
R₁₀ is absent, hydrogen, halogen, C1 to C4 alkyl, OH, OMe, OEt, CN, CF₃, NMe₂, piperazine, piperazine substituted with C1 to C3 alkyl, morpholine, or morpholine substituted with C1 to C3 alkyl.

The present inventors have confirmed that the novel compounds according to the present invention are effective for treatment of lung cancer, particularly, lung cancer in which an epidermal growth factor receptor mutation is shown. In particular, the compounds of the present invention are useful for treatment of lung cancer having cancer cells showing epidermal growth factor receptor (EGFR) EXON 20 Insertion mutation properties among lung cancer.

As used herein, the term "alkyl" means a saturated straight-chain or branched non-cyclic hydrocarbon having 1 to 10 carbon atoms (when the number of carbon atoms is not particularly limited). "Lower alkyl" refers to a straight-chain or branched alkyl having 1 to 4 carbon atoms. The representative saturated straight-chain alkyl comprises -methyl, -ethyl, -n-propyl, -n-butyl, -n-pentyl, -n-hexyl, -n-heptyl, -n-octyl, -n-nonyl and -n-decyl, while the saturated branched alkyl comprises -isopropyl, -sec-butyl, -isobutyl, -tert-butyl, isopentyl, 2-methylhexyl, 3-methylbutyl, 2-methylpentyl, 3-methylpentyl, 4-methylpentyl, 2-methylhexyl, 3-methylhexyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimethylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, and 3,3-diethylhexyl.

As used herein, the term "cycloalkyl" refers to a monocyclic or polycyclic saturated ring which has carbon and hydrogen atoms and does not have a carbon-carbon multiple bond. The example of the cycloalkyl group comprises (C₃-C₇)cycloalkyl (for example, cyclopropyl, cyclobutyl, cyclopentyl, cyclohexyl and cycloheptyl), but not limited thereto. In one aspect of the present invention, cycloalkyl is (C₃-C₅)cycloalkyl. In another aspect of the present invention, cycloalkyl is cyclopropyl. In one example, the cycloalkyl group is a monocyclic or bicyclic ring.

Herein, when described as "C₁₋₆" or "C1 to C6", this means that the number of carbon atoms is 1 to 6. For example, C₁₋₆ alkyl means alkyl having 1 to 6 carbon atoms.

As used herein, the term "halogen" and "halo" means fluorine, chlorine, bromine or iodine.

As used herein, the term "aryl" means a carbocyclic aromatic group containing 5 to 10 ring atoms. The representative example includes phenyl, tolyl, xylyl, naphthyl, tetrahydronaphthyl, anthracenyl, fluorenyl, indenyl, azulenyl, and the like, but not limited thereto. The carbocyclic aromatic group may be selectively substituted.

As used herein, "heteroaryl" is a 5- to 10-membered heterocyclic aromatic ring which has at least one heteroatom selected from the group consisting of nitrogen, oxygen and sulfur, and is a 5- to 10-membered heterocyclic aromatic ring including at least one carbon atom and having a mono- and bicyclic ring system. The representative heteroaryl is triazolyl, tetrazolyl, oxadiazolyl, pyridyl, furyl, benzofuranyl, thiophenyl, benzothiophenyl, quinolinyl, pyrrolyl, indolyl, oxazolyl, benzoxazolyl, imidazolyl, benzimidazolyl, thiazolyl, benzothiazolyl, isoxazolyl, pyrazolyl, isothiazolyl, pyridazinyl, pyrimidinyl, pyrazinyl, triazinyl, cinnolinyl, phthalazinyl, quinazolinyl, pyrimidyl, oxetanyl, azepinyl, piperazinyl, morpholinyl, dioxanyl, thietanyl and oxazolyl.

Herein, the compound represented by Chemical formula 1 may be used in a form of a salt induced by inorganic acid or organic acid, and for example, it may be used in a form of a salt induced by one or more kinds of acids selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, benzoic acid, hydroxybenzoic acid, phenylacetic acid, cinnamic acid, salicylic acid, methane sulfonic acid, ethane sulfonic acid, benzene sulfonic acid, toluene sulfonic acid, and the like.

As used herein, the term "compound of the present invention" means each compound of Chemical formula 1, as well as clathrates, hydrates, solvates or polymorphic forms thereof. In addition, the term "compound of the present invention" is a meaning comprising pharmaceutically acceptable salts of the compounds of the present invention, unless a pharmaceutically acceptable salt thereof is mentioned. In one example, the compound of the present invention may be present as stereoisomerically pure compounds (for example, substantially free of other stereoisomers (e.g., 85% ee or more, 90% ee or more, 95% ee or more, 97% ee or more, or 99% ee or more)). In other words, when the compound of Chemical formula 1 according to the present invention or a salt thereof is a tautomeric isomer and/or stereoisomer (e.g., geometrical isomer and conformational isomers), each of their isolated isomers and mixtures is also included within the scope of the compounds of the present invention. When the compound of the present invention or a salt thereof has an asymmetric carbon in its structure, their optically active compounds and racemic mixtures are also included within the scope of the compounds of the present invention.

As used herein, the term "polymorph" refers to a solid crystalline form of the compound of the present invention or a complex thereof. Different polymorphs of the same compound exhibit different physical, chemical and/or spectral properties. Differences in physical properties include stability (for example, thermal or light stability), compressibility and density (important for formulation and product preparation), and dissolution rate (which may affect bioavailability), but not limited thereto. Differences in stability cause changes in chemical reactivity (for example, differential oxidation such as faster discoloration when composed of one polymorph than when composed of another polymorph) or mechanical properties (for example, purified fragments stored as kinetically preferred polymorphs are converted to thermodynamically more stable polymorphs) or both of them (purification of one polymorph is more sensitive to degradation at high humidity). Other physical properties of the polymorph may affect their processing. For example, one polymorph may be more likely to form a solvate than another polymorph, for example, due to its shape or particle size distribution, or it may be more difficult to filter or wash.

As used herein, the term "solvent compound" refers to the compound of the present invention or pharmaceutically acceptable salt thereof comprising a stoichiometric or non-stoichiometric amount of a solvent combined by non-covalent intermolecular forces. Preferable solvents are volatile, non-toxic and may be administered in a trace amount to humans.

As used herein, the term "hydrate" refers to the compound of the present invention or pharmaceutically acceptable salt thereof comprising a stoichiometric or non-stoichiometric amount of water combined by non-covalent intermolecular forces.

As used herein, the term "clathrate" refers to the compound of the present invention or salt thereof in a crystal lattice form containing spaces (for example, channel) that confine a guest molecule (for example, solvent or water).

As used herein, the term "purified" means that the isolate is at least 90% pure, when isolated, and in one example, it means that it is 95% or more pure, and in another example, it means that it is 99% or more pure, and in other example, it means that it is 99.9% or more pure.

As used herein, "treatment" comprises eradication, removal, modification or control of primary, local or metastatic cancerous tissue; and minimizes or delays expansion of cancer.

The non-limitative, example of the compound according to the present invention comprises the following compounds and pharmaceutically acceptable salts thereof.
-N-(2-((5-chloro-2-((4-((2-(dimethylamino)ethyl)(methyl)amino)-5-(isopropylamino)-2-methoxyphenyl)amino)pyridin-4-yl)amino)phenyl)-N-methylsulfonic acid amide,
-N-(2-((2-((4-((2-(dimethylamino)ethyl)(methyl)amino)-5-(isopropylamino)-2-methoxy phenyl)amino)thieno[3,2-d]pyrimidin-4-yl)amino)phenyl)-N-methylmethanesulfonamide,
-N-(2-((2-((4-(4-(dimethylamino)piperidin-1-yl)-5-(isopropylamino)-2-methoxyphenyl)a mino)thieno[3,2-d]pyrimidin-4-yl)amino)phenyl)-N-methoxymethanesulfonamide,
-N-(2-((2-((5-(isopropylamino)-2-methoxy-4-(4-(4-methylpiperazin-1-yl)piperidin-1-yl) phenyl)amino)thieno[3,2-d]pyrimidin-4-yl)amino)phenyl)-N-methylmethanesulfonamide,
-N-(2-((5-chloro-2-((5-(isopropylamino)-2-methoxy-4-(4-(4-methylpiperazin-1-yl)piperi din-1-yl)phenyl)amino)pyrimidin-4-yl)amino)phenyl)-N-methylmethanesulfonamide,
-N-(2-((5-chloro-2-((4-(4-(dimethylamino)piperidin-1-yl)-5-(isopropylamino)-2-methox yphenyl)amino)pyrimidin-4-yl)amino)phenyl)-N-methylmethanesulfonamide,
-N-(4-methoxy-5-((4-((2-(N-methylmethylsulfonamido)phenyl)amino)thieno[3,2-d]pyri midin-2-yl)amino)-2-(4-(4-methylpiperazin-1-yl)piperidin-1-yl)phenyl)acrylamide,
-N-(2-((2-(dimethylamino)ethyl)(methyl)amino)-4-methoxy-5-((4-((2-(N-methylmethyls ulfonamido)phenyl)amino)thieno[3,2-d]pyrimidin-2-yl)amino)phenyl)acrylamide,
-N-(2-(4-(dimethylamino)piperidin-1-yl)-4-methoxy-5-((4-((2-(N-methylmethylsulfona mido)phenyl)amino)thieno[3,2-d]pyrimidin-2-yl)amino)phenyl)acrylamide,
-N-(5-((5-chloro-4-((2-(methylsulfonamido)phenyl)amino)pyrimidin-2-yl)amino)-2-((2-(dimethylamino)ethyl)(methyl)amino)-4-methoxyphenyl)acrylamide,
-N-(5-((5-chloro-4-((5-(N-methylmethylsulfonamido)quinoxalin-6-yl)amino)pyrimidin-2-yl)amino)-2-((2-(dimethylamino)ethyl)(methyl)amino)-4-methoxyphenyl)acrylamide,
-N-(5-((5-chloro-4-((5-(methylsulfonamido)quinoxalin-6-yl)amino)pyrimidin-2-yl)amin o)-2-((2-(dimethylamino)ethyl)(methyl)amino)-4-methoxyphenyl)acrylamide,
-N-(5-((5-chloro-4-((5-fluoro-2-(N-methylmethylsulfonamido)phenyl)amino)pyrimidin-2-yl)amino)-2-((2-(dimethylamino)ethyl)(methyl)amino)-4-methoxyphenyl)acrylamide,
-N-(5-((5-chloro-4-((5-fluoro-2-(methylsulfonamido)phenyl)amino)pyrimidin-2-yl)amin o)-2-((2-(dimethylamino)ethyl)(methyl)amino)-4-methoxyphenyl)acrylamide,
-isopropyl 2-((5-acrylamido-4-((2-(dimethylamino)ethyl)(methyl)amino)-2-methoxyphenyl)amino)-4-((2-( N-methylmethylsulfonamido)phenyl)amino)pyrimidine-5-carboxylate,
-cyclopropyl 2-((5-acrylamido-4-((2-(dimethylamino)ethyl)(methyl)amino)-2-methoxyphenyl)amino)-4-((2-( N-methylmethylsulfonamido)phenyl)amino)pyrimidine-5-carboxylate, or
N-(2-((2-(dimethylamino)ethyl)(methyl)amino)-4-methoxy-5-((6-((2-(N-methylmethyls ulfonamido)phenyl)amino)pyrimidin-4-yl)amino)phenyl)acrylamide.

In other aspect, the present invention provides a pharmaceutical composition comprising a therapeutically effective dose of the compound of Chemical formula 1 or a pharmaceutically acceptable salt thereof according to the present invention, and a pharmaceutically acceptable carrier.

As used herein, "effective dose" refers to an amount of the compound of the present invention sufficient to destroy, modify, control or remove primary, local or metastatic cancer cells or cancer tissue; slow or minimize expansion of cancer; or provide a therapeutic benefit in treatment or management of cancer, neoplastic disease or tumor. "Effective dose" also refers to an amount of the compound of the present invention sufficient to cause cancer or neoplastic cell death. "Effective dose" also refers to an amount sufficient to inhibit or reduce the activity of lung cancer cells, either in vitro or in vivo.

In other aspect, the present invention provides a method for treating a disease or condition comprising administering a therapeutically effective dose of the compound of Chemical formula 1 or a pharmaceutically acceptable salt thereof to a subject in need thereof, and the disease or condition is lung cancer. In other aspect, the subject is a human. In other aspect, the lung cancer is lung cancer showing epidermal growth factor receptor (EGFR) mutation properties. In other aspect, the lung cancer is lung cancer having cancer cells showing epidermal growth factor receptor EXON 20 Insertion mutation (EGFR EXON 20 Insertion mutation) properties.

In other words, the present invention provides a medical use characterized by using the compound of Chemical formula 1 or a pharmaceutically acceptable salt thereof according to the present invention as an active ingredient. In one aspect, the medical use of the present invention is a use for treatment or prevention of the disease or condition described herein.

Accordingly, in other aspect, the present invention provides a pharmaceutical composition for treatment or prevention of lung cancer, in particular, epidermal growth factor receptor mutation expressing lung cancer, comprising the compound according to the present invention or a pharmaceutically acceptable salt thereof as an active ingredient.

The compound of the present invention or a pharmaceutically acceptable salt thereof is administered generally in a therapeutically effective dose. The compound of the present invention may be administered by any appropriate route in an effective dose for the form of the pharmaceutical composition suitable for this route, and intended treatment. The effective dose may be generally about 0.0001 to about 200 mg/body weight kg/day, preferably, about 0.001 to about 100 mg/kg/day by single or divided administration. Depending on the age, species, and disease or condition to be treated, a dose level below the lower limit of this range may be suitable. In other cases, still larger doses may be used without deleterious side effects. The larger dose may be divided into several smaller doses for administration throughout the day. Methods for determining appropriate dose are well known in the art, and for example, the document Remington: The Science and Practice of Pharmacy, Mack Publishing Co., 20th ed., 2000 may be used.

For treatment of lung cancer, the compound or pharmaceutically acceptable salt thereof described herein may be administered by various methods as follows.

### Oral administration

The compound of the present invention may be administered orally, and oral is a concept comprising swallowing. By oral administration, the compound of the present invention may enter the gastrointestinal tract, or may be absorbed directly from the mouth into blood stream, for example, buccal or sublingual administration.

A composition suitable for oral administration may be in a solid, liquid, gel or powder form, and may have a formulation such as tablets, lozenges, capsules, granules, powders, and the like.

The composition for oral administration may be enteric coated selectively, and may exhibit delayed or sustained release through enteric coating. In other words, the composition for oral administration according to the present invention may be a formulation having an immediate or modified release pattern.

The liquid formulation may comprise solution, syrup and suspension, and this liquid composition may be a form contained in a soft or hard capsule. This formulation may comprise a pharmaceutically acceptable carrier, for example, water, ethanol, polyethylene glycol, cellulose or oil. The formulation may also comprise one or more emulsifying agents and/or suspending agents.

In a table formulation, the amount of the drug as an active ingredient may be present in an amount of about 0.05 % by weight to about 95 % by weight based on the total tablet weight, more generally, about 2 % by weight to about 50 % by weight of the formulation. In addition, the tablet may comprise a disintegrating agent comprised in an amount of about 0.5 % by weight to about 35 % by weight, more generally, about 2 % by weight to about 25 % by weight of the formulation. As the example of the disintegrating agent, lactose, starch, sodium starch glycolate, crospovidone, croscarmellose sodium, maltodextrin or mixtures thereof may be used, but not limited thereto.

An appropriate glydent comprised for preparation into a tablet may be present in an amount of about 0.1 % by weight to about 5 % by weight, and talc, silicon dioxide, stearic acid, calcium, zinc or magnesium stearate, sodium stearyl fumarate, and the like may be used as the glydent, but the present invention is not limited by these kinds of additives.

As a binder for preparation into a tablet, gelatin, polyethylene glycol, sugar, gum, starch, polyvinyl pyrrolidone, hydroxypropylcellulose, hydroxyproprylmethylcellulose, and the like may be used, and as a suitable diluent for preparation into a tablet, mannitol, xylitol, lactose, dextrose, sucrose, sorbitol, starch, microcrystalline cellulose and the like may be used, but the present invention is not limited by these kinds of additives.

A solubilizing agent which may be selectively comprised in a tablet may be used in an amount of about 0.1 % by weight to about 3 % by weight based on the total tablet weight, and for example, polysorbate, sodium laurylsulfate, sodium dodecylsulfate, propylene carbonate, diethylene glycol monoethylether, dimethyl isosorbide, polyoxyethyelene glycolated natural or hydrogenated castor oil, HCOR^{™} (Nikkol), oleyl ester, Gelucire^{™}, caprylic/caprylic acid mono/diglyceride, sorbitan fatty acid ester, Solutol HS^{™}, and the like may be used for the pharmaceutical composition according to the present invention, but the present invention is not limited by these specific kinds of solubilizing agents.

### Parenteral Administration

The compound of the present invention may be directly administered into bloodstream, muscle or intestine. A suitable method for parenteral administration comprises intravenous, intra-muscular, subcutaneous intraarterial, intraperitoneal, intrathecal, intracranial injection, and the like. A suitable device for parenteral administration comprises an injector (comprising needle and needleless injector) and an infusion method.

The composition for parenteral administration may be a formulation having an immediate or modified release pattern, and the modified release pattern may be a delayed or sustained release pattern.

Most parenteral formulations are liquid compositions, and these liquid compositions are aqueous solution comprising the medicinal ingredient according to the present invention, a salt, a buffer, an isotonic agent and the like.

Parenteral formulations may be also prepared in a dried form (for example, freeze dried) or sterile non-aqueous solution. These formulations may be used with a suitable vehicle such as sterile water. Solubility-enhancing agents may also be used in preparation of parenteral solution.

### Topical Administration

The compound of the present invention may be topically administered dermally or transdermally. Formulations for topical administration comprise lotion, solution, cream, gel, hydrogel, ointment, foam, implant, patch and the like. A pharmaceutically acceptable carrier for topical administration formulations may comprise water, alcohol, mineral oil, glycerin, polyethylene glycol, and the like. Topical administration may be also performed by electroporation, iontophoresis, phonophoresis, and the like.

The composition for topical administration may be a formulation having an immediate or modified release pattern, and the modified release pattern may be a delayed or sustained release pattern.

### [ADVANTAGEOUS EFFECTS]

The present invention provides new pyrimidine derivative compounds having cancer cell inhibitory activity and pharmaceutically acceptable salts thereof. The pyrimidine derivatives or pharmaceutically acceptable salts thereof according to the present invention can effectively inhibit growth of epidermal growth factor receptor mutation expressing cancer cells, particularly, cancer cells present in lung cancer. Accordingly, the compounds and pharmaceutically acceptable salts thereof according to the present invention are useful for treatment of lung cancer.

### [MODE FOR INVENTION]

Hereinafter, to help understanding of the present invention, it will be described in detail by examples and the like. However, the examples according to the present invention may be modified in various other forms, and the scope of the present invention should not be construed as being limited to the following examples. The examples of the present invention are provided to more completely describe the present invention to those with average knowledge in the art to which the present invention pertains.

The compounds represented by Chemical formula 1 above according to the present invention may be easily prepared, for example, by referring to the methods represented by Reaction schemes 1, 2, 3 and 4 below.

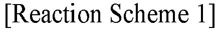

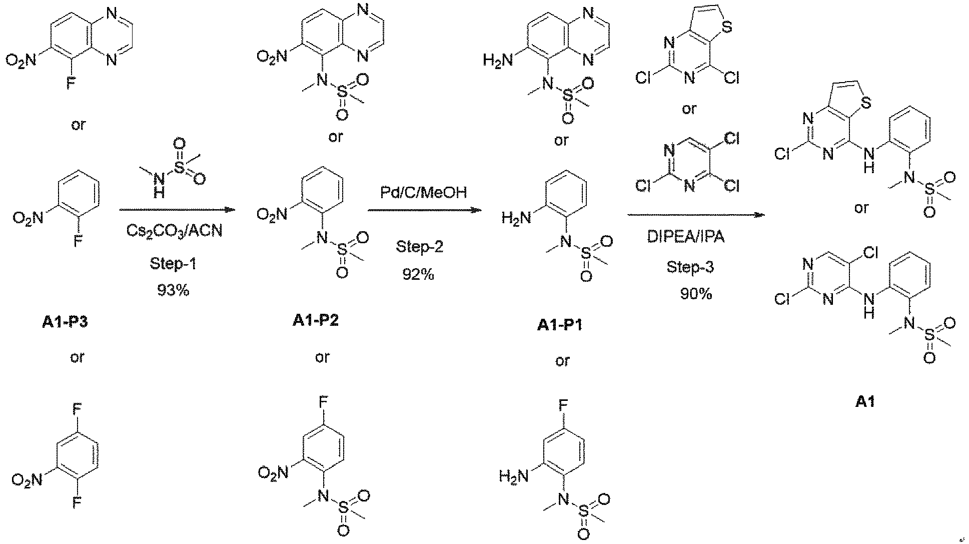

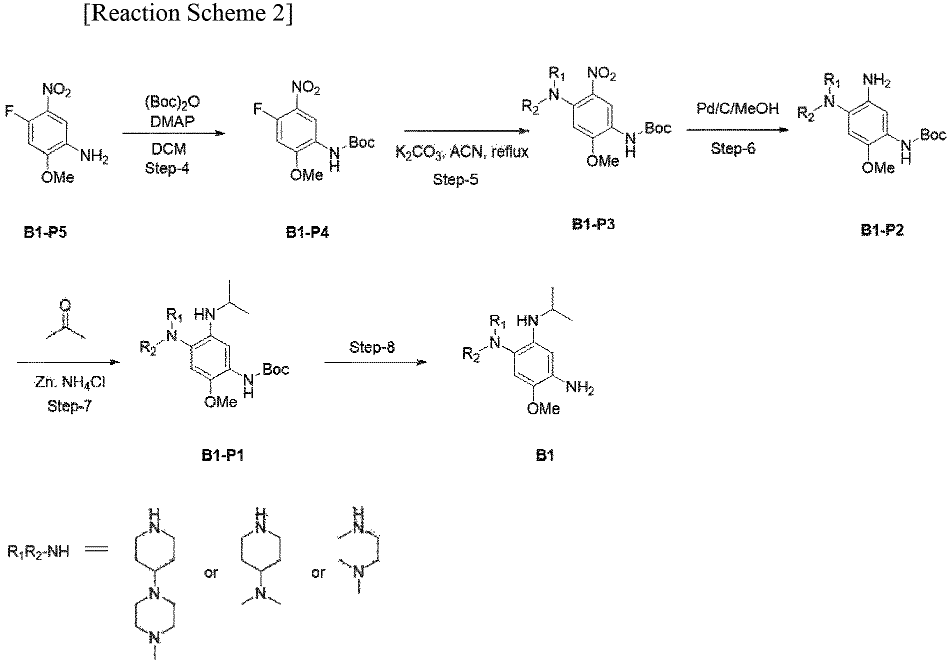

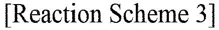

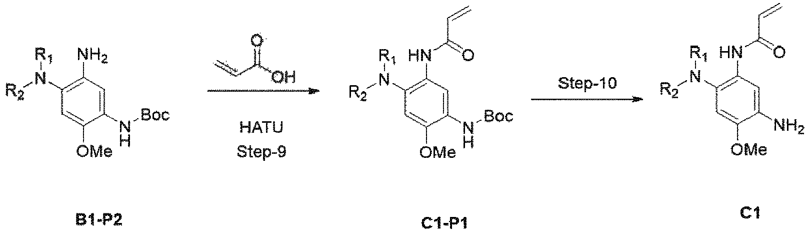

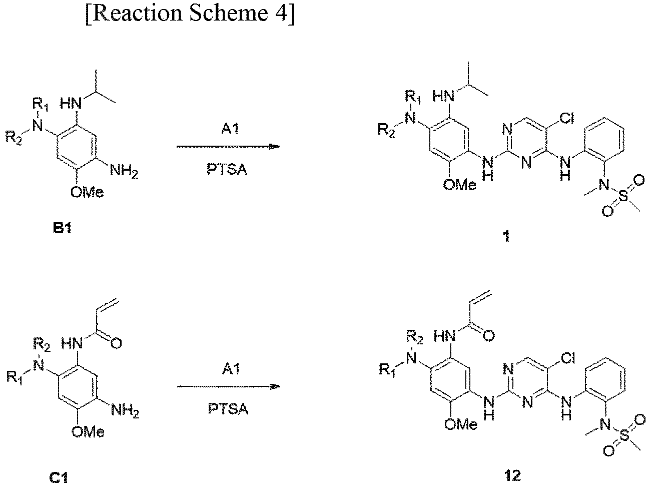

**Synthetic example:** Synthesis of N-(2-((5-chloro-2-((4-((2-(dimethylamino)ethyl)(methyl)amino)-5-(isopropylamino)-2-methoxy phenyl)amino)pyridin-4-yl)amino)phenyl)-N-methylsulfonic acid amide and N-(5-((5-chloro-4-((2-(N-methylmethylsulfonamido)phenyl)amino)pyrimidin-2-yl)amino)-2-((2-(dimethylamino)ethyl)(methyl)amino)-4-methoxyphenyl)acrylamide

### Step-1:

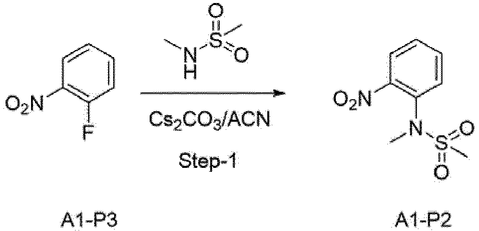

In Reaction Scheme 1 above, the structure A1-P3 (3g, 21.262mmol) was dissolved with acetonitrile (150mL). At a room temperature, CS₂CO₃ (10.4g, 31.892mmol) and N-methylmethanesulfonamide were added, and then it was stirred at 80 °C for 12 hours. The solid remaining after the reaction was filtered, and the organic solvent was distilled under reduced pressure to remove it, and then Al-P2 was obtained. Without a separate separation process, it was used for the next reaction.

### Step-2:

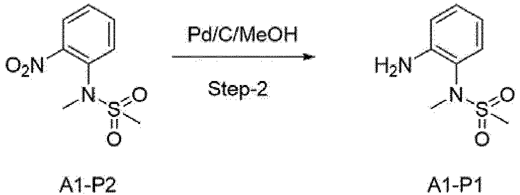

The structure A1-P2 (4.5g, 19.545mmol) obtained in the Step-1 was dissolved in a mixed solvent of MeOH (100mL) and DCM (50mL), and then 10% Pd/C (0.416g, 3.909mmol) was added. Under the hydrogen environment, it was stirred for 2 hours and then filtered using celite, and it was distilled under reduced pressure to remove the solvent to obtain crude A1-P1. This solid was washed with ether and n-pentane and used for the next reaction without a separate separation process.

### Step-3:

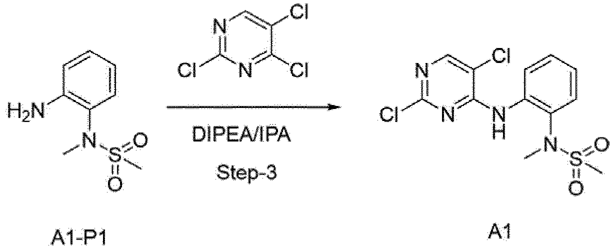

The structure A1-P1 (8.3g, 41.446mmol) obtained in the previous reaction, was dissolved in IPA (200mL), and then at a room temperature, 2,5,6-trichloropyrimidine (12.163g, 66.314mmol) and DIPEA (21.428g, 166mmol) were added, and it was stirred at 90 °C for 12 hours. When the reaction was completed, it was distilled under reduced pressure to remove the solvent, and then water was added and it was extracted with DCM. The mixed solution was washed with 2N HCl again, and the organic layer was separated and distilled under reduced pressure to obtain crude A1. Without a separate separation process, it was used for the next reaction.

### Step-4:

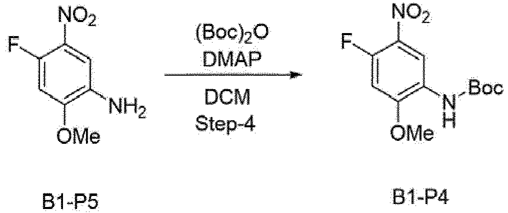

In Reaction Scheme 2 above, the structure B1-P5 (4g, 21.489mmol) was dissolved in DCM (100mL), and then at a room temperature, Boc anhydride (4.690g, 21.489mmol) and DMAP (0.262g, 2.149mmol) were added, and it was stirred for 12 hours. When the reaction was completed, the reaction mixture was washed with 2N HCl, and then dried with Na₂SO₄ and filtered, and then distilled under reduced pressure to obtain crude B1-P4. Using column chromatography, purified pure B1-P4 target compound (5.8g, 97.5%) was obtained.

### Step-5:

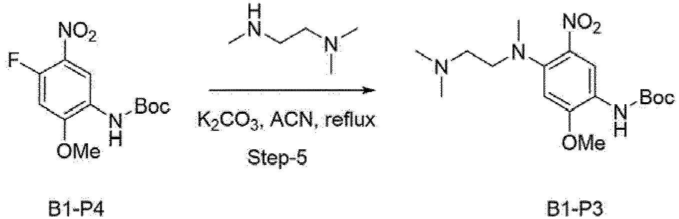

In Reaction Scheme 2 above, the structure B1-P4 (2.2g, 7.685mmol) was dissolved in acetonitrile (50mL), and then at a room temperature, K₂CO₃ (2.124g, 15.371mmol) and N,N,N'-trimethylenediamine were added, and then it was stirred at 80 °C for 12 hours. When the reaction was completed, the reacted products were filtered and distilled under reduced pressure to obtain crude B1-P3. Without a separate separation process, it was used for the next reaction.

### Step-6:

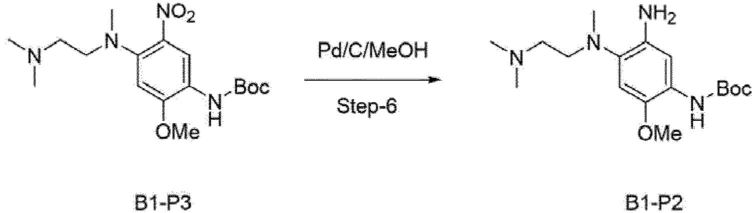

In Reaction Scheme 2 above, the structure B1-P3 (2.8g, 7.6mmol) was dissolved in a mixed solvent of MeOH (50mL) and DCM (20mL) and it was stirred under the hydrogen environment for 4 hours. When the reaction was completed, it was filtered using celite and the solvent was removed by distillation under reduced pressure to obtain crude B1-P2. Without a separate separation process, it was used for the next reaction.

### Step-7:

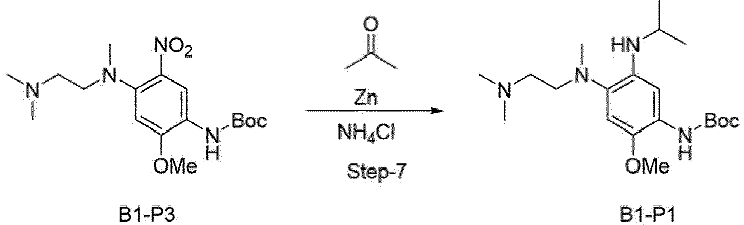

In Reaction Scheme 2 above, acetone 20ml was added to B1-P3 (580.8 mg, 1.00 mmol) and zinc powder (653.9 mg, 10.0 mmol) and sat. aq. NH₄Cl(10mL) were added, and the reaction was completed under reflux for 6 hours. After cooling at a room temperature, zinc powder was removed using celite and the solvent was removed under reduced pressure, and then the obtained water layer was extracted using EtOAc. In the obtained organic layer, the solvent was removed under reduced pressure, and it was purified using column chromatography.

### Step-8:

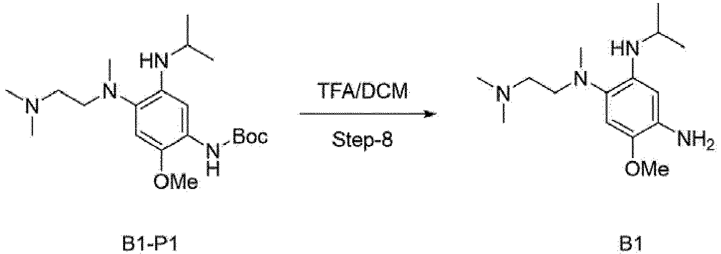

In Reaction Scheme 2 above, B1-P1 (300mg, 0.764mmol) was dissolved in DCM (15mL) and then cooled to 0 °C. Then, TFA (0.6mL, 7.643mmol) was added, and the temperature was increased to a room temperature and it was stirred for 3 hours. When the reaction was completed, it was distilled under reduced pressure to remove the solvent, and it was washed with DCM and sat. NaHCO₃ solution, and then the organic layer was separated and dried, and then distilled under reduced pressure. Without a separate separation process, it was used for the next reaction.

### Step-9:

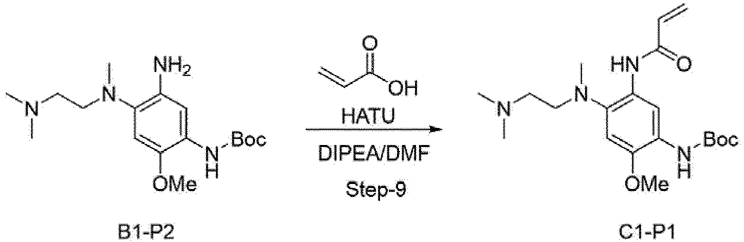

In Reaction Scheme 3 above, Acrylic acid (75mg, 1.034mmol) was dissolved in DMF (4mL) and then cooled to 0 °C. Then, HATU (393mg, 1.034mmol) and DIPEA (400mg, 3.103mmol) were added, and then B1-P2 (350mg, 1.034mmol) was added, and the reactants were stirred at a room temperature for 12 hours. When the reaction was completed, it was extracted with H₂O and DCM. The organic layer was separated and dried with Na₂SO₄, and filtered and then distilled under reduced pressure. Using column chromatography, purified pure B1-P1 target compound (220mg, 55%) was obtained.

### Step-10:

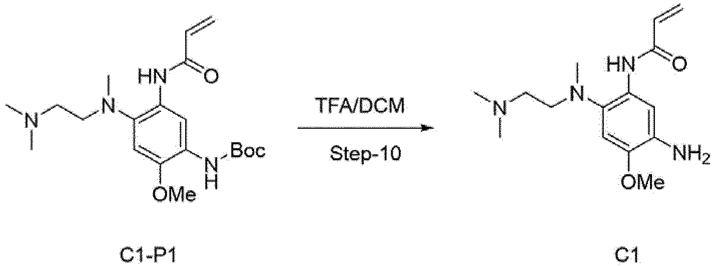

In Reaction Scheme 3 above, C1-P1 (300mg, 0.764mmol) was dissolved in DCM (15mL) and then cooled to 0 °C. TFA (0.6mL, 7.643mmol) was added and the temperature was increased to a room temperature and it was stirred for 3 hours. When the reaction was completed, it was distilled under reduced pressure to remove the solvent and washed with DCM and sat. NaHCO₃ solution, and then the organic layer was separated and dried and then distilled under reduced pressure. Without a separate separation process, it was used for the next reaction.

### Step-11:

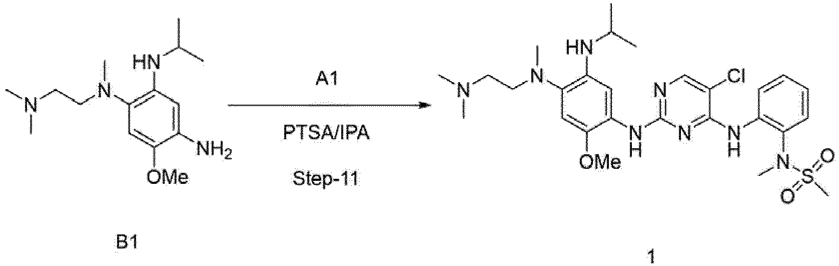

In Reaction Scheme 4 above, the structure B1 (50mg, 0.178mmol) was dissolved in isopropyl alcohol (3mL) and then A1 (71mg, 0.205mmol) and PTSA (48.8mg, 0.257mmol) were added at a room temperature, and it was stirred at 90 °C for 12 hours. When the reaction was completed, it was distilled under reduced pressure to remove the solvent, and it was extracted with water and 10% MeOH/DCM. The organic layer was separated and dried, and distilled under reduced pressure to obtain crude Compound 1. Using column chromatography, purified pure Compound 1 (60mg, 58%) was obtained.

¹H NMR (400 MHz; CDCl3): δ (ppm): 8.26-8.49 (2H, m), 8.16 (1H, s) 8.09-8.08 (1H, d), 7.83 (1H, s), 7.55-7.52 (1H, dd), 6.98 (1H, s), 6.97-6.77 (1H, dd), 5.72 (1H, dd), 4.76 (1H, m), 3.67 (3H, s), 3.14 (3H, s), 3.06 (3H, s), 2.79 (2H, m), 2.70 (3H, s), 2.20 (8H, br s), 1.11 (6H, d). [M + H]⁺: m/z 591.17 found 592.2 HPLC Purity: 97.9%

### Step-12:

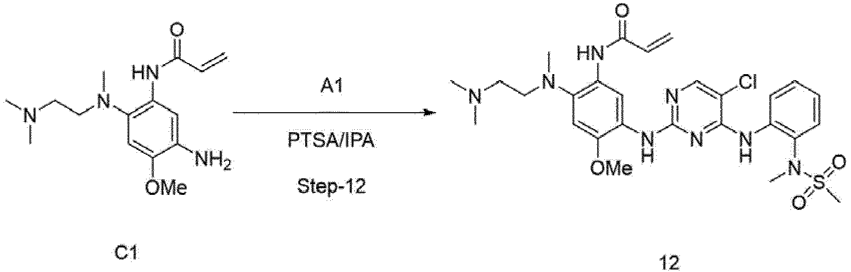

In Reaction Scheme 4 above, the structure C1 (50mg, 0.171mmol) was dissolved in isopropyl alcohol (3mL) and then A1 (71mg, 0.205mmol) and PTSA (49.1mg, 0.258mmol) were added at a room temperature, and it was stirred at 90 °C for 12 hours. When the reaction was completed, it was distilled under reduced pressure to remove the solvent, and it was extracted with water and 10% MeOH/DCM. The organic layer was separated and dried, and distilled under reduced pressure to obtain crude Compound 12. Using column chromatography, purified pure Compound 12 (65mg, 63%) was obtained.

¹H NMR (400 MHz; CDCl3): δ (ppm): 10.02 (1H, br s), 8.35-8.31 (2H, m), 8.26 (1H, s) 8.21-8.19 (1H, d), 8.09 (1H, s), 7.53-7.50 (1H, dd), 6.95 (1H, s), 6.18-6.14 (1H, dd), 5.72-5.68 (1H, dd), 4.31 (1H, m), 3.72 (3H, s), 3.14 (3H, s), 3.05 (3H, s), 2.86 (2H, m), 2.66 (3H, s), 2.19 (8H, br s), 1.12 (6H, d). [M + H]⁺: m/z 591.17 found 592.2 HPLC Purity: 98.1%

### Example 1. Preparation of N-(2-((5-chloro-2-((4-((2-(dimethylamino)ethyl)(methyl)amino)-5-(isopropylamino)-2-meth oxyphenyl)amino)pyridin-4-yl)amino)phenyl)-N-methylsulfonic acid amide

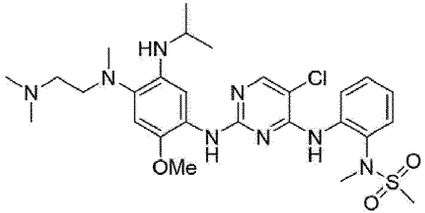

The final compound was prepared by Reaction Scheme 4 above.

¹H NMR (400 MHz; CDCl3): δ (ppm): 8.26-8.49 (2H, m), 8.16 (1H, s) 8.09-8.08 (1H, d), 7.83 (1H, s), 7.55-7.52 (1H, dd), 6.98 (1H, s), 6.97-6.77 (1H, dd), 5.72 (1H, dd), 4.76 (1H, m), 3.67 (3H, s), 3.14 (3H, s), 3.06 (3H, s), 2.79 (2H, m), 2.70 (3H, s), 2.20 (8H, br s), 1.11 (6H, d). [M + H]⁺: m/z 591.17 found 592.2 HPLC Purity: 97.9%

### Example 2. Preparation of N-(2-((2-((4-((2-(dimethylamino)ethyl)(methyl)amino)-5-(isopropylamino)-2-methoxypheny l)amino)thieno[3,2-d]pyrimidin-4-yl)amino)phenyl)-N-methylmethanesulfonamide

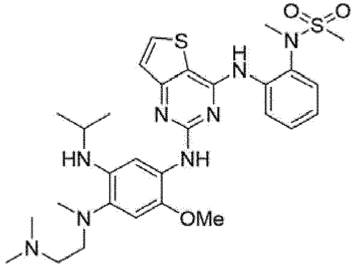

The final compound was prepared by Reaction Scheme 4 above.

1H NMR (400 MHz, DMSO-d6) δ 9.51 (s, 1H), 9.20 (s, 1H), 8.02 (s, 1H), 7.63 (d, J = 7.6 Hz, 1H), 7.45 (dd, J = 7.5, 1.5 Hz, 1H), 7.39 - 7.32 (m, 2H), 7.04 (td, J = 7.5, 1.5 Hz, 1H), 6.76 (td, J = 7.5, 1.5 Hz, 1H), 6.46 (s, 1H), 5.51 (d, J = 8.4 Hz, 1H), 4.31 (m, 1H), 3.85 (s, 3H), 3.44 - 3.34 (bs, 2H), 3.32 (s, 3H), 3.26 (s, 3H), 3.00 (s, 3H), 2.57-2.50 (m, 8H), 1.12 (d, J = 6.9 Hz, 6H). MS: ESI m/z 613.1 [M+H]+ HPLC Purity: 99.0%

### Example 3. Preparation of N-(2-((2-((4-(4-(dimethylamino)piperidin-1-yl)-5-(isopropylamino)-2-methoxyphenyl)amino )thieno[3,2-d]pyrimidin-4-yl)amino)phenyl)-N-methoxymethanesulfonamide

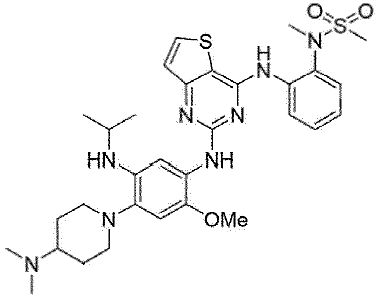

The final compound was prepared by Reaction Scheme 4 above.

1H NMR (400 MHz, DMSO-d6) δ 9.51 (s, 1H), 9.20 (s, 1H), 8.02 (s, 1H), 7.63 (d, J = 7.6 Hz, 1H), 7.45 (dd, J = 7.5, 1.5 Hz, 1H), 7.39 - 7.32 (m, 2H), 7.04 (td, J = 7.5, 1.5 Hz, 1H), 6.76 (td, J = 7.5, 1.5 Hz, 1H), 6.46 (s, 1H), 5.51 (d, J = 8.4 Hz, 1H), 4.31 (m, 1H), 3.72 (s, 3H), 3.14 (s, 3H), 3.06 (s, 3H), 3.00 (d, J = 11.2 Hz, 2H), 2.62 (dd, J = 12.4, 10.2 Hz, 2H), 2.19 (m, 8H), 1.80 (d, J = 11.4 Hz, 2H), 1.65 (q, J = 11.3 Hz, 2H), 1.12 (d, J = 6.9 Hz, 6H). MS: ESI m/z 639.0 [M+H]+ HPLC Purity: 98.5%

### Example 4. Preparation of N-(2-((2-((5-(isopropylamino)-2-methoxy-4-(4-(4-methylpiperazin-1-yl)piperidin-1-yl)phen yl)amino)thieno[3,2-d]pyrimidin-4-yl)amino)phenyl)-N-methylmethanesulfonamide

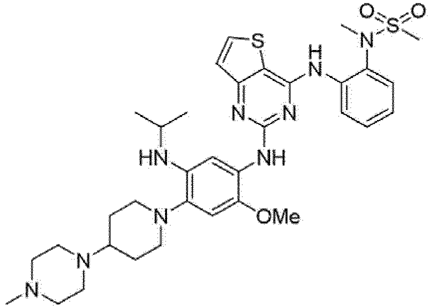

The final compound was prepared by Reaction Scheme 4 above.

1H NMR (400 MHz, DMSO-d6) δ 9.50 (s, 1H), 9.22 (s, 1H), 8.00 (s, 1H), 7.61 (d, J = 7.6 Hz, 1H), 7.45 (dd, J = 7.5, 1.5 Hz, 1H), 7.37 - 7.33 (m, 2H), 7.04 (td, J = 7.5, 1.5 Hz, 1H), 6.76 (td, J = 7.5, 1.5 Hz, 1H), 6.46 (s, 1H), 5.51 (d, J = 8.4 Hz, 1H), 4.32 (m, 1H), 3.70 (s, 3H), 3.15 (s, 3H), 3.04 (s, 3H), 3.01 (d, J = 11.2 Hz, 2H), .69 - 2.57 (m, 2H), 2.55 - 2.48 (m, 4H), 2.25 (m, 5H), 2.12 (s, 3H), 1.81 (d, J = 11.9 Hz, 2H), 1.67 (q, J = 11.4, 10.7 Hz, 2H), 1.10 (d, J = 6.9 Hz, 6H). MS: ESI m/z 694.1 [M+H]+ HPLC Purity: 97.9%

### Example 5. Preparation of N-(2-((5-chloro-2-((5-(isopropylamino)-2-methoxy-4-(4-(4-methylpiperazin-1-yl)piperidin-1 -yl)phenyl)amino)pyrimidin-4-yl)amino)phenyl)-N-methylmethanesulfonamide

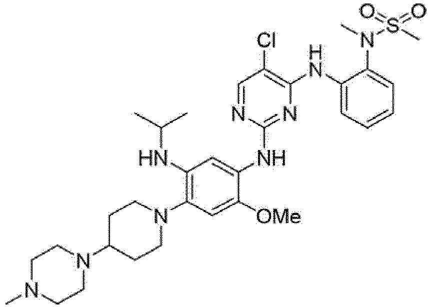

The final compound was prepared by Reaction Scheme 4 above.

1H NMR (400 MHz; CDCl3): δ (ppm): 10.02 (1H, br s), 8.35-8.31 (2H, m), 8.26 (1H, s) 8.21-8.19 (1H, d), 8.09 (1H, s), 7.53-7.50 (1H, dd), 6.95 (1H, s), 6.18-6.14 (1H, dd), 5.72-5.68 (1H, dd), 4.31 (1H, m), 3.72 (s, 3H), 3.14 (s, 3H), 3.06 (s, 3H), 3.00 (2H, d), 2.69 - 2.57 (2H, m), 2.55 - 2.48 (4H, m), 2.25 (5H, m), 2.12 (3H, s), 1.81 (2H, d), 1.67 (2H, q), 1.12 (d, 6H). MS: ESI m/z 672.0 [M+H]+ HPLC Purity: 98.2%

### Example 6. Preparation of N-(2-((5-chloro-2-((4-(4-(dimethylamino)piperidin-1-yl)-5-(isopropylamino)-2-methoxyphe nyl)amino)pyrimidin-4-yl)amino)phenyl)-N-methylmethanesulfonamide

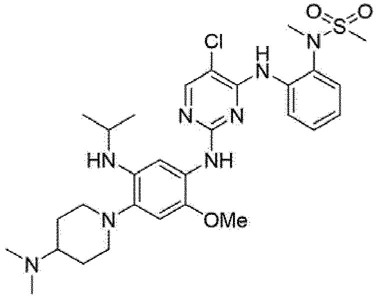

The final compound was prepared by Reaction Scheme 4 above.

1H NMR (400 MHz; CDCl3): δ (ppm): 10.02 (1H, br s), 8.37-8.33 (2H, m), 8.23 (1H, s) 8.20-8.17 (1H, d), 8.09 (1H, s), 7.53-7.50 (1H, dd), 6.95 (1H, s), 6.18-6.14 (1H, dd), 5.72-5.68 (1H, dd), 4.31 (1H, m), 3.72 (s, 3H), 3.14 (s, 3H), 3.06 (s, 3H), 3.00 (d, J = 11.2 Hz, 2H), 2.62 (2H, dd), 2.19 (8H, m), 1.80 (2H, d), 1.65 (2H, q), 1.12 (6H, d). MS: ESI m/z 617.0 [M+H]+ HPLC Purity: 97.6%

### Example 7. Preparation of N-(4-methoxy-5-((4-((2-(N-methylmethylsulfonamido)phenyl)amino)thieno[3,2-d]pyrimidin -2-yl)amino)-2-(4-(4-methylpiperazin-1-yl)piperidin-1-yl)phenyl)acrylamide

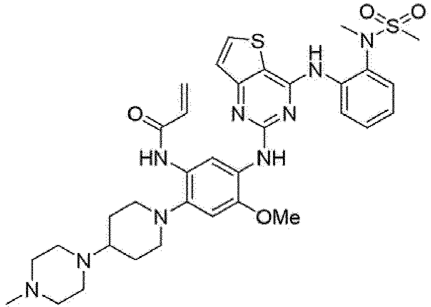

The final compound was prepared by Reaction Scheme 4 above.

1H NMR (400 MHz, DMSO-d6) δ 10.05 (s, 1H), 8.75 (s, 1H), 8.36 (s, 1H), 8.03 (d, J = 5.3 Hz, 1H), 8.00 (dd, J = 8.1, 1.6 Hz, 1H), 7.70 (s, 1H), 7.52 (dd, J = 7.9, 1.6 Hz, 1H), 7.24 (td, J = 7.7, 1.6 Hz, 1H), 7.20 - 7.11 (m, 2H), 6.93 (s, 1H), 6.37 (m, 1H), 6.18 (dd, J = 16.9, 2.1 Hz, 1H), 5.74 - 5.66 (m, 1H), 3.72 (s, 3H), 3.14 (s, 3H), 3.06 (s, 3H), 3.00 (d, J = 11.2 Hz, 2H), 2.69 - 2.57 (m, 2H), 2.55 - 2.48 (m, 4H), 2.25 (m, 5H), 2.12 (s, 3H), 1.81 (d, J = 11.9 Hz, 2H), 1.67 (q, J = 11.4, 10.7 Hz, 2H). MS: ESI m/z 706.2 [M+H]+, HPLC Purity: 98.5%.

### Example 8. Preparation of N-(2-((2-(dimethylamino)ethyl)(methyl)amino)-4-methoxy-5-((4-((2-(N-methylmethylsulfon amido)phenyl)amino)thieno[3,2-d]pyrimidin-2-yl)amino)phenyl)acrylamide

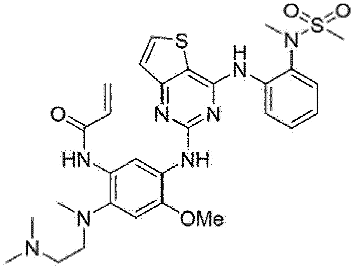

The final compound was prepared by Reaction Scheme 4 above.

1H NMR (400 MHz, DMSO-d6) δ 10.03 (s, 1H), 8.74 (s, 1H), 8.36 (s, 1H), 8.03 (d, J = 5.3 Hz, 1H), 8.00 (dd, J = 8.1, 1.6 Hz, 1H), 7.71 (s, 1H), 7.52 (dd, J = 7.9, 1.6 Hz, 1H), 7.24 (td, J = 7.7, 1.6 Hz, 1H), 7.20 - 7.11 (m, 2H), 6.93 (s, 1H), 6.37 (m, 1H), 6.18 (dd, J = 16.9, 2.1 Hz, 1H), 5.74 - 5.66 (m, 1H), 3.75 (s, 3H), 3.13 (s, 3H), 3.01 (s, 3H), 2.84 (bs, 2H), 2.65 (s, 3H), 2.37 - 2.07 (m, 8H). MS: ESI m/z 625.1 [M+H]+, HPLC Purity: 97.9%.

### Example 9. Preparation of N-(2-(4-(dimethylamino)piperidin-1-yl)-4-methoxy-5-((4-((2-(N-methylmethylsulfonamido) phenyl)amino)thieno[3,2-d]pyrimidin-2-yl)amino)phenyl)acrylamide

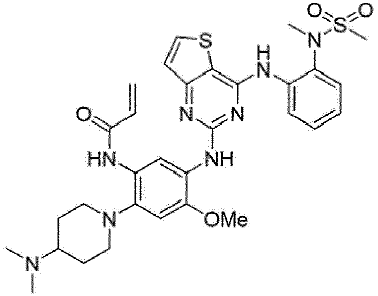

The final compound was prepared by Reaction Scheme 4 above.

1H NMR (400 MHz, DMSO-d6) δ 10.05 (s, 1H), 8.74 (s, 1H), 8.35 (s, 1H), 8.03 (d, J = 5.3 Hz, 1H), 8.00 (dd, J = 8.1, 1.6 Hz, 1H), 7.70 (s, 1H), 7.52 (dd, J = 7.9, 1.6 Hz, 1H), 7.24 (td, J = 7.7, 1.6 Hz, 1H), 7.20 - 7.11 (m, 2H), 6.93 (s, 1H), 6.37 (m, 1H), 6.18 (dd, J = 16.9, 2.1 Hz, 1H), 5.74 - 5.66 (m, 1H), 3.72 (s, 3H), 3.14 (s, 3H), 3.06 (s, 3H), 3.00 (d, J = 11.2 Hz, 2H), 2.62 (dd, J = 12.4, 10.2 Hz, 2H), 2.19 (s, 7H), 1.80 (d, J = 11.4 Hz, 2H), 1.65 (q, J = 11.3 Hz, 2H). MS: ESI m/z 650.0 [M+H]+, HPLC Purity: 98.1%.

### Example 10. Preparation of N-(5-((5-chloro-4-((2-(methylsulfonamido)phenyl)amino)pyrimidin-2-yl)amino)-2-((2-(dime thylamino)ethyl)(methyl)amino)-4-methoxyphenyl)acrylamide

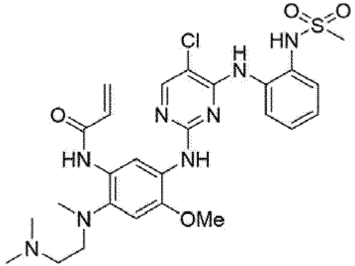

The final compound was prepared by Reaction Scheme 4 above.

1H NMR(400 MHz; DMSO-d6): δ (ppm): 10.02 (1H, br s), 8.35-8.31 (2H, m), 8.26 (1H, s) 8.21-8.19 (1H, d), 8.09 (1H, s), 7.53-7.50 (1H, dd), 7.12-7.04 (2H, m), 6.95 (1H, s), 6.37 (1H, br s), 6.18-6.14 (1H, dd), 5.72-5.68 (1H, dd), 3.72 (3H, s), 3.14 (3H, s), 2.86 (2H, m), 2.66 (3H, s), 2.19 (8H, br s). [M + H]⁺: m/z 588.20, found 589.1 HPLC Purity: 98.2%

### Example 11. Preparation of N-(5-((5-chloro-4-((5-(N-methylmethylsulfonamido)quinoxalin-6-yl)amino)pyrimidin-2-yl)a mino)-2-((2-(dimethylamino)ethyl)(methyl)amino)-4-methoxyphenyl)acrylamide

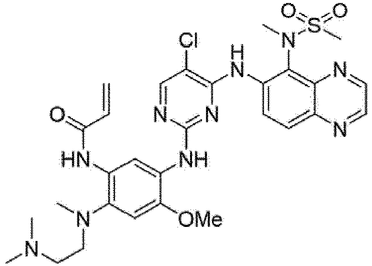

The final compound was prepared by Reaction Scheme 4 above.

1H NMR (400 MHz, DMSO-d6) δ 9.51 (d, J = 7.3 Hz, 2H), 9.43 (s, 1H), 9.34 (d, J = 7.5 Hz, 1H), 8.58 (d, J = 7.5 Hz, 1H), 8.52 (s, 1H), 7.58 (d, J = 7.5 Hz, 1H), 7.53 (d, J = 7.3 Hz, 2H), 6.41 (s, 1H), 6.34 (dd, J = 16.7, 10.0 Hz, 1H), 6.03 (dd, J = 10.0, 3.1 Hz, 1H), 5.93 (dd, J = 16.7, 3.1 Hz, 1H), 3.72 (s, 3H), 3.14 (s, 3H), 3.05 (s, 3H), 2.86 (m, 2H), 2.66 (s, 3H), 2.19 (m, 8H). MS: ESI m/z 655.1 [M+H]+, HPLC Purity: 98.7%

### Example 12. Preparation of N-(5-((5-chloro-4-((5-(methylsulfonamido)quinoxalin-6-yl)amino)pyrimidin-2-yl)amino)-2-( (2-(dimethylamino)ethyl)(methyl)amino)-4-methoxyphenyl)acrylamide

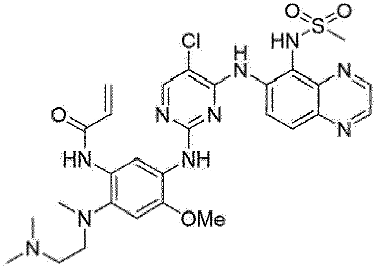

The final compound was prepared by Reaction Scheme 4 above.

1H NMR (400 MHz, DMSO-d6) δ 9.50 (d, J = 7.3 Hz, 2H), 9.43 (s, 1H), 9.36 (d, J = 7.5 Hz, 1H), 8.58 (d, J = 7.5 Hz, 1H), 8.52 (s, 1H), 7.56 (d, J = 7.5 Hz, 1H), 7.53 (d, J = 7.3 Hz, 2H), 6.41 (s, 1H), 6.34 (dd, J = 16.7, 10.0 Hz, 1H), 6.03 (dd, J = 10.0, 3.1 Hz, 1H), 5.93 (dd, J = 16.7, 3.1 Hz, 1H), 3.72 (s, 3H), 3.12 (s, 3H), 2.86 (m, 2H), 2.66 (s, 3H), 2.19 (m, 8H). MS: ESI m/z 641.0 [M+H]+, HPLC Purity: 97.3%.

### Example 13. Preparation of N-(5-((5-chloro-4-((5-fluoro-2-(N-methylmethylsulfonamido)phenyl)amino)pyrimidin-2-yl) amino)-2-((2-(dimethylamino)ethyl)(methyl)amino)-4-methoxyphenyl)acrylamide

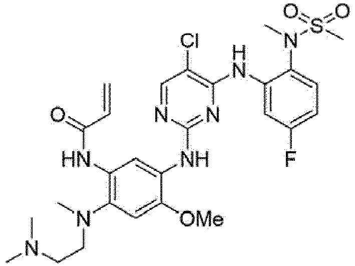

The final compound was prepared by Reaction Scheme 4 above.

1H NMR (400 MHz, DMSO-d6) δ 9.49 (s, 1H), 9.42 (d, J = 8.2 Hz, 2H), 8.52 (s, 1H), 7.75 (dd, J = 8.0, 1.5 Hz, 1H), 7.54 (s, 1H), 7.31 (dd, J = 7.5, 5.0 Hz, 1H), 6.93 - 6.84 (m, 1H), 6.41 (s, 1H), 6.34 (dd, J = 16.7, 10.0 Hz, 1H), 6.03 (dd, J = 10.0, 3.1 Hz, 1H), 5.93 (dd, J = 16.7, 3.1 Hz, 1H), 3.72 (s, 3H), 3.14 (s, 3H), 3.05 (s, 3H), 2.86 (bs, 2H), 2.63 (s, 3H), 2.20 (m, 8H). MS: ESI m/z 621.0 [M+H]+ HPLC Purity: 98.9%.

### Example 14. Preparation of N-(5-((5-chloro-4-((5-fluoro-2-(methylsulfonamido)phenyl)amino)pyrimidin-2-yl)amino)-2-((2-(dimethylamino)ethyl)(methyl)amino)-4-methoxyphenyl)acrylamide

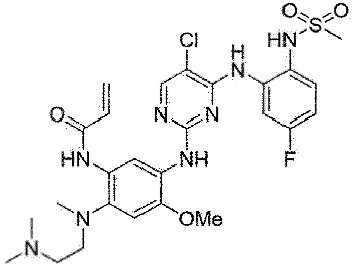

The final compound was prepared by Reaction Scheme 4 above.

1H NMR (400 MHz, DMSO-d6) δ 9.67 (s, 1H), 9.54 (s, 1H), 9.43 (d, J = 2.5 Hz, 2H), 8.52 (s, 1H), 7.56 - 7.48 (m, 3H), 6.84 (ddd, J = 8.0, 7.5, 1.5 Hz, 1H), 6.41 (s, 1H), 6.34 (dd, J = 16.7, 10.0 Hz, 1H), 6.03 (dd, J = 10.0, 3.1 Hz, 1H), 5.93 (dd, J = 16.7, 3.1 Hz, 1H), 3.70 (s, 3H), 3.16 (s, 3H), 2.83 (bs, 2H), 2.63 (s, 3H), 2.18 (m, 8H). MS: ESI m/z 607.0 [M+H]+ HPLC Purity: 98.7%.

### Example 15. Preparation of isopropyl 2-((5-acrylamido-4-((2-(dimethylamino)ethyl)(methyl)amino)-2-methoxyphenyl)amino)-4-(( 2-(N-methylmethylsulfonamido)phenyl)amino)pyrimidine-5-carboxylate

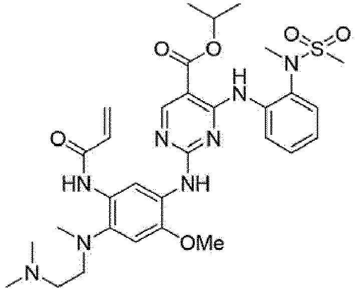

The final compound was prepared by Reaction Scheme 4 above.

1H NMR (400 MHz, DMSO-d6) δ 10.22 (s, 1H), 9.59 - 9.48 (m, 2H), 8.84 (s, 1H), 7.54 (s, 1H), 7.18 (dd, J = 7.4, 1.5 Hz, 1H), 7.08 - 6.99 (m, 2H), 6.95 (td, J = 7.1, 2.1 Hz, 1H), 6.41 (s, 1H), 6.34 (dd, J = 16.7, 10.1 Hz, 1H), 6.03 (dd, J = 9.9, 3.1 Hz, 1H), 5.93 (dd, J = 16.7, 3.1 Hz, 1H), 5.09 (h, J = 6.2 Hz, 1H), 3.81 (s, 3H), 3.26 (t, J = 6.8 Hz, 2H), 3.12 (s, 3H), 3.08 (s, 3H), 2.83 (s, 3H), 2.43 (m, 2H), 2.11 (s, 6H), 1.28 (d, J = 6.2 Hz, 6H), MS: ESI m/z 655.0 [M+H]+ HPLC Purity: 98.9%.

### Example 16. Preparation of cyclopropyl 2-((5-acrylamido-4-((2-(dimethylamino)ethyl)(methyl)amino)-2-methoxyphenyl)amino)-4-(( 2-(N-methylmethylsulfonamido)phenyl)amino)pyrimidine-5-carboxylate

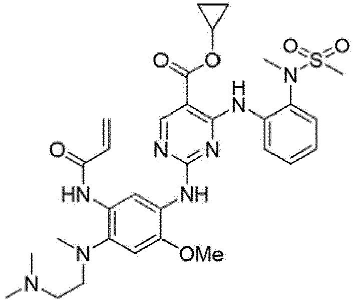

The final compound was prepared by Reaction Scheme 4 above.

1H NMR (400 MHz, DMSO-d6) δ 10.20 (s, 1H), 9.58 - 9.47 (m, 2H), 8.82 (s, 1H), 7.54 (s, 1H), 7.18 (dd, J = 7.4, 1.5 Hz, 1H), 7.08 - 6.99 (m, 2H), 6.95 (td, J = 7.1, 2.1 Hz, 1H), 6.41 (s, 1H), 6.34 (dd, J = 16.7, 10.1 Hz, 1H), 6.03 (dd, J = 9.9, 3.1 Hz, 1H), 5.93 (dd, J = 16.7, 3.1 Hz, 1H), 5.06 (m, 1H), 3.79 (s, 3H), 3.26 (t, J = 6.8 Hz, 2H), 3.15 (s, 3H), 3.09 (s, 3H), 2.80 (s, 3H), 2.43 (m, 2H), 2.11 (s, 6H), 0.98-0.95 (m, 2H), 0.88-0.85 (m, 2H), MS: ESI m/z 653.0 [M+H]+ HPLC Purity: 98.6%.

### Synthetic example of Example 17

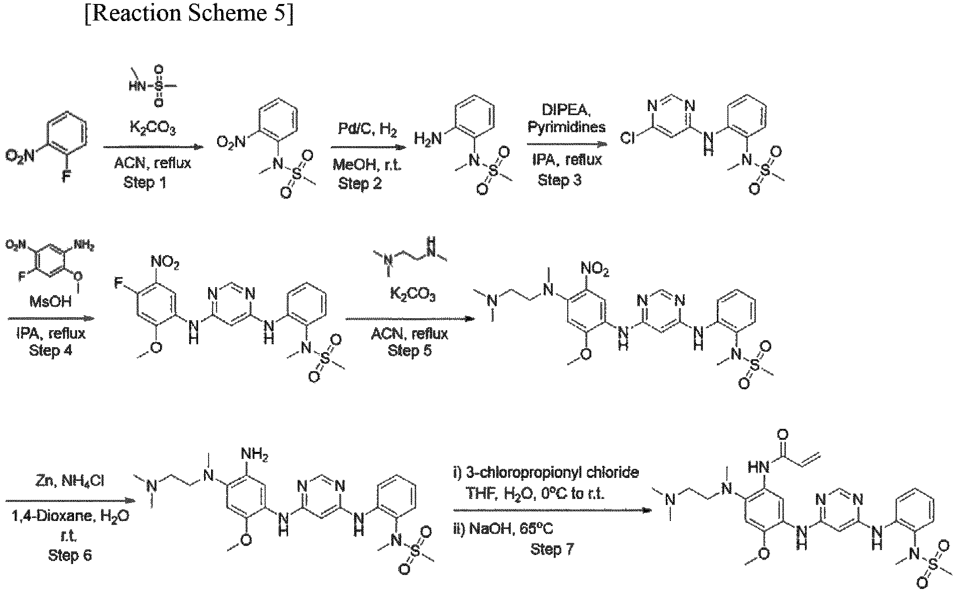

### Step-1: Synthesis of N-methyl-N-(2-nitrophenyl)methanesulfonamide

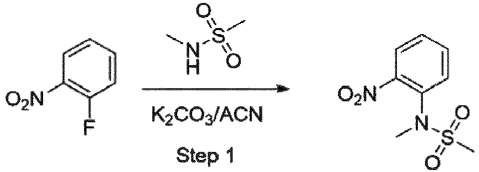

1-Fluoro-2-nitrobenzene (1.0 eq.) was dissolved in acetonitrile and potassium carbonate (2.0 eq.) and *N*-methylmethanesulfonamide (1.4 eq.) were added at a room temperature. Then, it was stirred at 80°C overnight. After completing the reaction, the temperature was lowed to a room temperature and it was filtered. The filtrate was evaporated under reduced pressure to obtain a compound. Without a separation process, it was used for the next reaction.

### Step-2: Synthesis of N-(2-aminophenyl)-N-methylmethanesulfonamide

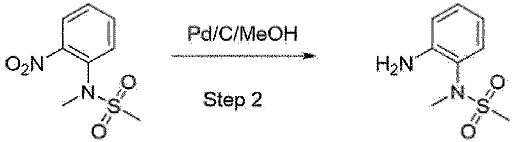

*N*-methyl-*N*-(2-nitrophenyl)methanesulfonamide (1.0 eq.) was dissolved in methanol and ethyl acetate (1:1) and 10% palladium/charcoal (0.2 eq.) was added. Under hydrogen, it was stirred for 2 hours. After completing the reaction, it was filtered by using celite and then the filtrate was evaporated under reduced pressure. Using ethyl ether and pentane, it was solidified and filtered to obtain a target compound. Without a separation process, it was used for the next reaction.

### Step-3: Synthesis of N-(2-((6-chloropyrimidin-4-yl)amino)phenyl)-N-methylmethanesulfonamide

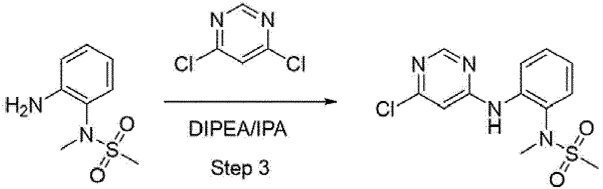

*N*-(2-aminophenyl)-*N*-methylmethanesulfonamide (1.0 eq.) was dissolved in isopropylalcohol, and 2,4,5-trichloropydirine (1.1 eq.) and N,N-diisopropylethylamine (2.5 eq.) were added at a room temperature. It was stirred at 80°C overnight. After completing the reaction, it was evaporated under reduced pressure and it was extracted using water and dichloromethane. The organic layer was washed using 2N hydrochloric acid. The organic layer was evaporated under reduced pressure and column chromatograph was conducted to obtain a target compound. (50% hexane/ethyl acetate)

### Step-4: Synthesis of N-(2-((6-((4-fluoro-2-methoxy-5-nitrophenyl)amino)pyrimidin-4-yl)amino)phenyl)-N-methylmethanesulfonamide

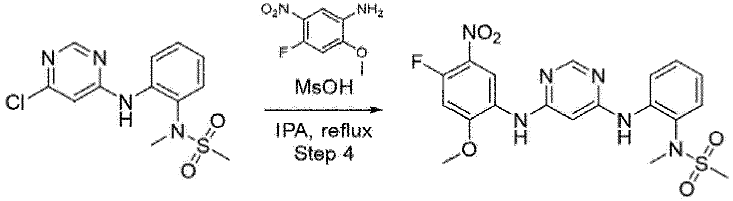

A pyrimidine derivative (1.0 eq.) was dissolved in isopropyl alcohol and an aniline derivative (1.0 eq.) and methane sulfonic acid (1.3 eq.) were added at a room temperature. It was stirred at 80°C overnight. After completing the reaction, it was evaporated under reduced pressure and it was extracted using a mixed solution of water and 10% methanol/dichloromethane. The organic layer was evaporated under reduced pressure and column chromatograph was conducted to obtain a target compound. (50% hexane/ethyl acetate)

### Step-5: Synthesis of N-(2-((6-((4-((2-(dimethylamino)ethyl)(methyl)amino)-2-methoxy-5-nitrophenyl)amino)pyrimidin-4-yl)amino)phenyl)-N-methylmethanesulfonamide

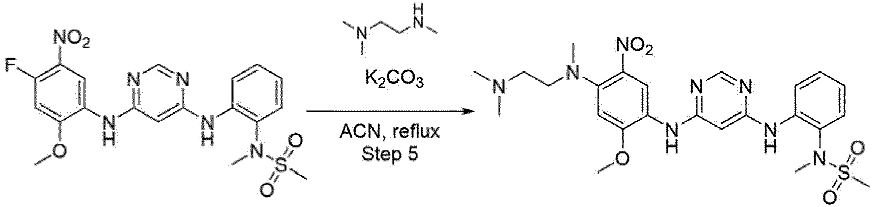

N-(2-((6-((4-fluoro-2-methoxy-5-nitrophenyl)amino)pyrimidin-4-yl)amino)phenyl)-N-m ethylmethanesulfonamide (1.0 eq.) was dissolved in acetonitrile and potassium carbonate (3.0 eq.) and amine chain (1.2 eq.) were added at a room temperature. It was stirred overnight by reflux. After completing the reaction, the temperature was lowered to a room temperature and it was filtered. The filtrate was evaporated under reduced pressure to obtain a target compound. Without a separation process, it was used for the next reaction.

### Step-6: Synthesis of N-(2-((6-((5-amino-4-((2-(dimethylamino)ethyl)(methyl)amino)-2-methoxyphenyl)amino)py rimidin-4-yl)amino)phenyl)-N-methylmethanesulfonamide

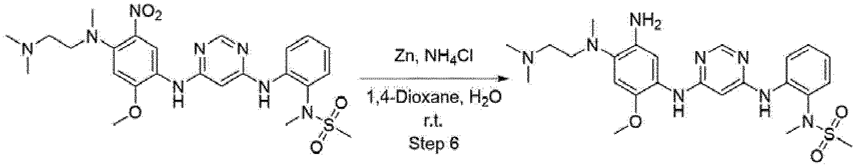

N-(2-((6-((4-((2-(dimethylamino)ethyl)(methyl)amino)-2-methoxy-5-nitrophenyl)amino )pyrimidin-4-yl)amino)phenyl)-N-methylmethanesulfonamide (1.0 eq.) was dissolved in 1,4-dioxane, and zinc (10.0 eq.) and ammonium chloride (10.0 eq.) were added at a room temperature, and it was stirred overnight. After completing the reaction, the temperature was lowered to a room temperature, and after celite filtering, the filtrate was evaporated under reduced pressure to obtain a compound. Without a separation process, it was used for the next reaction.

### Step-7: Synthesis of N-(2-((2-(dimethylamino)ethyl)(methyl)amino)-4-methoxy-5-((6-((2-(N-methylmethylsulfon amido)phenyl)amino)pyrimidin-4-yl)amino)phenyl)acrylamide

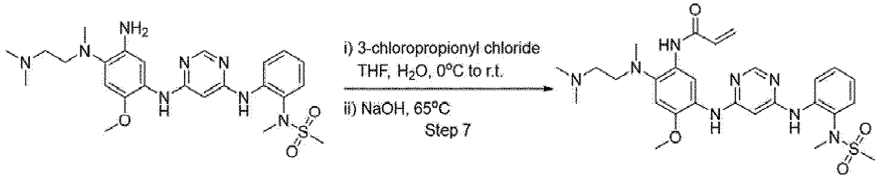

N-(2-((6-((5-amino-4-((2-(dimethylamino)ethyl)(methyl)amino)-2-methoxyphenyl)amin o)pyrimidin-4-yl)amino)phenyl)-N-methylmethanesulfonamide (1.0 eq.) was dissolved in tetrahydrofuran and water, and 3-chloropropionyl chloride (1.2 eq.) was added at 0±5°C. At the same temperature, it was stirred for 15 minutes. After completing the reaction, sodium hydroxide (4.0 eq.) was added at the same temperature. By increasing the reactor temperature, it was stirred at 65°C overnight. After completing the reaction, the solvent was removed by evaporation under reduced pressure and it was extracted using a mixed solution of water and 10% methanol/dichloromethane. The organic layer was evaporated under reduced pressure and column chromatography was conducted to obtain a target compound. (10% methyl alcohol/dichloromethane)

### Example 17. N-(2-((2-(dimethylamino)ethyl)(methyl)amino)-4-methoxy-5-((6-((2-(N-methylmethylsulfon amido)phenyl)amino)pyrimidin-4-yl)amino)phenyl)acrylamide

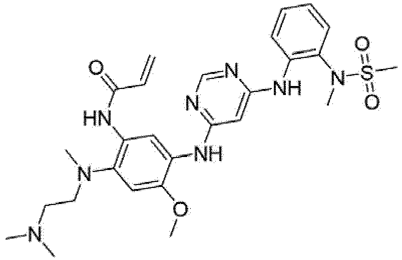

Yield : 23.0%, White solid, 1H NMR (400 MHz, DMSO-d6) δ 10.04 (s, 1H), 8.42 (s, 1H), 8.29 (s, 1H), 8.09 (d, J = 0.9 Hz, 1H), 8.00 (s, 1H), 7.79 (dd, J = 8.2, 1.5 Hz, 1H), 7.46 (dd, J = 8.0, 1.5 Hz, 1H), 7.26 (ddd, J = 8.4, 7.4, 1.6 Hz, 1H), 7.07 (td, J = 7.6, 1.5 Hz, 1H), 6.93 (s, 1H), 6.34 (dd, J = 16.9, 10.0 Hz, 1H), 6.19 (dd, J = 16.9, 2.2 Hz, 1H), 6.04 (d, J = 1.1 Hz, 1H), 5.71 (dd, J = 10.0, 2.2 Hz, 1H), 3.74 (s, 3H), 3.10 (s, 3H), 2.99 (s, 3H), 2.80 (t, J = 5.9 Hz, 2H), 2.66 (s, 3H), 2.26 (t, J = 5.8 Hz, 2H), 2.16 (s, 6H). MS: ESI m/z 569.14 [M+H]+

### Experimental example 1: Measurement of inhibitory effect on cancer cell growth

Since lung cancer cell line having an EGFR insertion mutation cannot be available commercially, a method for inserting 3-4 amino acids at each site in a wild type EGFR expression vector using site direct mutagenesis was attempted. First, the present invention used a vector in which NPG of 3 amino acids were added to D770_N771 site of the most frequent alterations, and used the Ba/F3 cell line for expression of these genes. The Ba/F3 cell line is Murine IL3-dependent pro B cell line showing cell growth only when IL-3 is added, and has mutant EGFR dependency on cell growth and death in an absence of IL-3, when an oncogenic mutant EGFR is expressed. Because substances showing effective inhibitory effect on the mutant EGFR inhibit cell growth and induce apoptosis, the anti-cancer effect on cells was analyzed by MTT assay.

Pre-constructed stable cells of 1 X 10⁴ cells were placed in a 96 well plate and incubated overnight, and then the compounds of Examples were treated at a dose dependent manner. After 72 hours, MTT reagent was added, and after 3 hours, stop buffer (10% SDS) was added. After 24 hours of incubation, the result was analyzed by reading at 595nm, and the IC₅₀ value was calculated at a concentration where each compound inhibited cell growth by 50%. The result was shown as A, B, C, and D in Table 1 below. Herein, A means IC₅₀≤100nM, and B means IC₅₀ =100-300nM, and C means IC₅₀ = 300-1,000nM, and D means IC₅₀>1,000nM. As a control drug, Osimertinib was used.

Measured value of inhibitory effect on cancer cell growth (GI₅₀)

**[Table 1]**

| Example | EXON 20 Insertion (NPG) Ba/F3 Cell |
|---|---|
| 1 | C |
| 2 | C |
| 3 | C |
| 4 | C |
| 5 | C |
| 6 | C |
| 7 | B |
| 8 | B |
| 9 | B |
| 10 | A |
| 11 | A |
| 12 | A |
| 13 | A |
| 14 | A |
| 15 | A |
| 16 | A |
| 17 | A |
| Osimertinib | C |

As shown in Table 1, the compounds according to the present invention exhibited excellent activity to lung cancer cell line expressing the EXON 20 Insertion mutation of epidermal growth factor receptor. In particular, the activity of Examples 10-17 compounds was more excellent. On the other hand, the activity of the control drug, Osimertinib was relatively weak.

Accordingly, the present invention suggests a novel pyrimidine derivative which can treat lung cancer expressing the EXON 20 Insertion mutation in the epidermal growth factor receptor.

## Claims

1. A compound of Chemical formula 1 below or a pharmaceutically acceptable salt thereof: 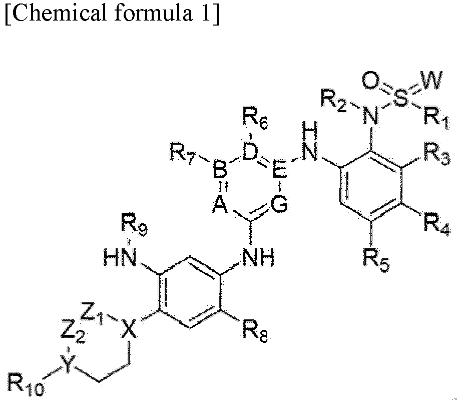 In the Chemical formula 1,
A, B, D, E, and G are combinations of A=N, B=C, D=C, E=C, and G=N, or combinations of A=N, B=C, D=N, E=C, and G=C,
W is oxygen or NH,
X and Y are each independently CH, oxygen or nitrogen,
Z₁ and Z₂ are each independently C1 to C4 alkyl, or are each independently composed of carbon atom and are linked to each other to form a 5-, 6- or 7-membered ring together with X and Y,
Z₁ and Z₂ are each independently not present when X or Y is oxygen, or are each independently C1 to C4 alkyl, or are each independently composed of carbon atom and are linked to each other to form a 5-, 6- or 7-membered ring together with X and Y, when X or Y is not oxygen,
R₁ is C1 to C4 alkyl,
R₂ is hydrogen, or C1 to C4 alkyl,
R₃ and R₄ are each independently hydrogen, halogen, OH, OMe, OEt, CN, CF₃, C1 to C4 alkyl, or are linked to each other to form a 5- or 6-membered heteroaryl ring,
R₅ is hydrogen, halogen, OH, OMe, OEt, CN, CF₃, or C1 to C4 alkyl,
R₆ and R₇ are each independently hydrogen, halogen, OH, OMe, OEt, CN, CF₃, COOH, COO-C₁₋₄ alkyl, COO-C₁₋₅ cycloalkyl, C1 to C4 alkyl, or are linked to each other to form a 5- or 6-membered heteroaryl ring,
R₈ is hydrogen, halogen, OH, OMe, OEt, CN, CF₃, or C1 to C4 alkyl,
R₉ is -C(O)-CH=CH₂ or C1 to C4 alkyl, and
R₁₀ is absent, hydrogen, halogen, C1 to C4 alkyl, OH, OMe, OEt, CN, CF₃, NMe₂, piperazine, piperazine substituted with C1 to C3 alkyl, morpholine, or morpholine substituted with C1 to C3 alkyl.

2. The compound or pharmaceutically acceptable salt thereof according to claim 1, wherein the compound is
N-(2-((5-chloro-2-((4-((2-(dimethylamino)ethyl)(methyl)amino)-5-(isopropylamino)-2-methoxyphenyl)amino)pyridin-4-yl)amino)phenyl)-N-methylsulfonic acid amide,
N-(2-((2-((4-((2-(dimethylamino)ethyl)(methyl)amino)-5-(isopropylamino)-2-methoxyp henyl)amino)thieno[3,2-d]pyrimidin-4-yl)amino)phenyl)-N-methylmethanesulfonamide,
N-(2-((2-((4-(4-(dimethylamino)piperidin-1-yl)-5-(isopropylamino)-2-methoxyphenyl)a mino)thieno[3,2-d]pyrimidin-4-yl)amino)phenyl)-N-methoxymethanesulfonamide,
N-(2-((2-((5-(isopropylamino)-2-methoxy-4-(4-(4-methylpiperazin-1-yl)piperidin-1-yl)p henyl)amino)thieno[3,2-d]pyrimidin-4-yl)amino)phenyl)-N-methylmethanesulfonamide,
N-(2-((5-chloro-2-((5-(isopropylamino)-2-methoxy-4-(4-(4-methylpiperazin-1-yl)piperi din-1-yl)phenyl)amino)pyrimidin-4-yl)amino)phenyl)-N-methylmethanesulfonamide,
N-(2-((5-chloro-2-((4-(4-(dimethylamino)piperidin-1-yl)-5-(isopropylamino)-2-methoxy phenyl)amino)pyrimidin-4-yl)amino)phenyl)-N-methylmethanesulfonamide,
N-(4-methoxy-5-((4-((2-(N-methylmethylsulfonamido)phenyl)amino)thieno[3,2-d]pyri midin-2-yl)amino)-2-(4-(4-methylpiperazin-1-yl)piperidin-1-yl)phenyl)acrylamide,
N-(2-((2-(dimethylamino)ethyl)(methyl)amino)-4-methoxy-5-((4-((2-(N-methylmethyls ulfonamido)phenyl)amino)thieno[3,2-d]pyrimidin-2-yl)amino)phenyl)acrylamide,
N-(2-(4-(dimethylamino)piperidin-1-yl)-4-methoxy-5-((4-((2-(N-methylmethylsulfonam ido)phenyl)amino)thieno[3,2-d]pyrimidin-2-yl)amino)phenyl)acrylamide,
N-(5-((5-chloro-4-((2-(methylsulfonamido)phenyl)amino)pyrimidin-2-yl)amino)-2-((2-( dimethylamino)ethyl)(methyl)amino)-4-methoxyphenyl)acrylamide,
N-(5-((5-chloro-4-((5-(N-methylmethylsulfonamido)quinoxalin-6-yl)amino)pyrimidin-2 -yl)amino)-2-((2-(dimethylamino)ethyl)(methyl)amino)-4-methoxyphenyl)acrylamide,
N-(5-((5-chloro-4-((5-(methylsulfonamido)quinoxalin-6-yl)amino)pyrimidin-2-yl)amino )-2-((2-(dimethylamino)ethyl)(methyl)amino)-4-methoxyphenyl)acrylamide,
N-(5-((5-chloro-4-((5-fluoro-2-(N-methylmethylsulfonamido)phenyl)amino)pyrimidin-2 -yl)amino)-2-((2-(dimethylamino)ethyl)(methyl)amino)-4-methoxyphenyl)acrylamide,
N-(5-((5-chloro-4-((5-fluoro-2-(methylsulfonamido)phenyl)amino)pyrimidin-2-yl)amino )-2-((2-(dimethylamino)ethyl)(methyl)amino)-4-methoxyphenyl)acrylamide,
isopropyl 2-((5-acrylamido-4-((2-(dimethylamino)ethyl)(methyl)amino)-2-methoxyphenyl)amino)-4-((2-( N-methylmethylsulfonamido)phenyl)amino)pyrimidine-5-carboxylate,
cyclopropyl 2-((5-acrylamido-4-((2-(dimethylamino)ethyl)(methyl)amino)-2-methoxyphenyl)amino)-4-((2-( N-methylmethylsulfonamido)phenyl)amino)pyrimidine-5-carboxylate, or
N-(2-((2-(dimethylamino)ethyl)(methyl)amino)-4-methoxy-5-((6-((2-(N-methylmethyls ulfonamido)phenyl)amino)pyrimidin-4-yl)amino)phenyl)acrylamide.

3. A composition comprising the compound or pharmaceutically acceptable salt of claim 1 or claim 2, and a pharmaceutically acceptable carrier.

4. A pharmaceutical composition for treatment or prevention of lung cancer, **characterized by** comprising the compound or pharmaceutically acceptable salt of claim 1 or claim 2 as an active ingredient.

5. The pharmaceutical composition for treatment or prevention of lung cancer according to claim 4, wherein the lung cancer is lung cancer expressing epidermal growth factor receptor mutation.

6. The pharmaceutical composition for treatment or prevention of lung cancer according to claim 4, wherein the mutation is EXON 20 Insertion mutation.
